# EUROPEAN PATENT SPECIFICATION

(11) **EP 4 467 030 B1**
(45) Date of publication and mention of the grant of the patent: **25.03.2026**
(21) Application number: 24177196.3
(22) Date of filing: 22.05.2024
(51) Int. Cl.: A41D 1/04, A41D 13/12, A41D 27/02, A61H 7/00

(54) **A GARMENT FOR PROVIDING SENSORY STIMULATION TO THE BODY OF A USER**
KLEIDUNGSSTÜCK ZUR BEREITSTELLUNG VON SENSORISCHER STIMULATION FÜR DEN KÖRPER EINES BENUTZERS
VÊTEMENT POUR FOURNIR UNE STIMULATION SENSORIELLE AU CORPS D'UN UTILISATEUR

(30) Priority: 22.05.2023 DK PA202330038
(43) Date of publication of application: 27.11.2024
(73) Proprietor: ZiboCare A/S, 8700 Horsens (DK)
(72) Inventor: DENKER, Jeannette B., 8700 Horsens (DK)
(74) Representative: Rasmussen, Martin Hoffgaard

(56) References cited:
- CN-U- 204 232 323
- US-A- 5 860 163
- ANONYMOUS PROTAC: "Protac MyFit - Sansestimulerende kuglevest", 4 February 2023 (2023-02-04), XP093335998, Retrieved from the Internet <URL:https://web.archive.org/web/20230204213521/https://protac.dk/dk/myfit> [retrieved on 20251114]

## Description

### Field of the invention

The present invention relates to the field of therapeutic wearables for providing tactile stimulation to the human body.

More specifically, the present invention relates in a first aspect to a garment for providing tactile sensory stimulation to the torso of a user.

In a second aspect, the present invention relates to a garment according to the first aspect of the invention for use in therapy against psychiatric conditions.

### Background of the invention

The sensory system or tactile system of the skin of the human body provides via receptors in the skin input to the brain relating to temperature, pressure, touch and pain and thereby information is provided to the brain helping the human individual of perceiving the surrounding.

Besides this, upon registering touch or pressure applied to the skin, the sensory system or tactile system of the skin of the human body also provides a more subtle effect of releasing certain hormones in the brain, such as oxytocin.

Oxytocin has important physical and psychological effects, including influencing social behavior and emotion. Oxytocin affects bonding behavior, the creation of group memories and social recognition.

People suffering from psychiatric conditions, such as Attention Deficit Hyperactivity Disorder (ADHD), Sensory Processing Disorder (SPD), disorders belonging to the autism spectrum, depression and anxiety etc. may feel and behave physically restless and cognitive unfocused.

These conditions may accordingly imply that the individual becomes hyperactive, occupied with his or her inner mental world and hence socially isolated.

It is well known in the art that the above conditions may be alleviated with different psycho-active pharmaceutical compounds.

However, most psycho-active pharmaceutical compounds present various more or less severe side-effects, and therefore it is desirably to avoid such medical treatment.

It is also well known in the art that the above conditions may also be alleviated by making the individual wear a specially designed garment having a plurality of ball-shaped weight elements embodied therein.

This type of garment usually comprises an inner lining and an outer fabric. Between the inner lining and the outer fabric, the ball shaped weight elements are arranged.

An example of such a garment, according to the preamble of claim 1, is marketed as the Myfit^{®} by the company Protac (https://protac.dk/dk/myfit). The diameter of the ball shaped elements in this product is either 2.5 cm or 3.8 cm.

The ball-shaped weight elements will provide pressure to the skin of the torso of the individual, as the individual moves around and this applied pressure will have an alleviating and calming effect of the individual, as explained above.

Although the effect of such type of weighted garment has been well-documented and hence indeed will alleviate certain mental disorders, such weighted garments have not found widespread use.

One reason for this may be that as the weight elements are ball-shaped and are being present right under the outer fabric of the garment, the ball-shaped weight elements form a rather curvy outer surface of the garment. Hereby, it is inevitably that other persons than the wearer of the garment easily recognizes the garment as being of a "special design" that one has never seen before.

Such a "special design" of a weighted garment may accordingly lead to a not insignificant stigmatization of the wearer and in case of kids wearing such garment even bullying or mocking of the wearer.

For this reason, the "special design" of the above type of weighted garment seems to lead individuals suffering from one of the above disorders, to avoid treatment using such a weighted garment.

US 5,860,163 discloses a heat insulating garment, such as in the form of a jacket. The jacket may comprise an outer layer, a thermal lining thereunder which comprises beads, and under this lining, an inner layer comprising a moist barrier. Underneath the moist barrier is arranged a lining. The disclosed garments are particularly intended for use by firefighters or as clothing for protection against cold. The beads are semi-spheric and a convex surface thereof may either face inward or outward. According to US 5,860,163 the orientation of the convex surface of the beads is not critical. The function of the beads is to create an air gap between two layers of fabric in such a way that the beads create thermal insulation. There is no disclosure in US 5,860,163 that the beads may have a tactile stimulating effect on the wearer of the garment.

CN 204 232 323 U discloses a detachable shoulder massaging garment for massaging shoulders to people doing office work at a desk. The garment comprises a garment body with patches which are arranged on an outside surface of the garment at the position of the shoulders on the garment body. The patches are disclosed as being magnetic and heat generating. The patches may comprise semispherical protrusions made from rubber. The protrusions are arranged on an outside surface of the garment. The protrusions point towards the shoulder of the wearer in order to provide massage to the shoulders. CN 204 232 323 U does not disclose that other parts of the garment are being provided with semi-spheric protrusions, nor that the protrusions are arranged between sheets of fabric. CN 204 232 323 U neither discloses the use of such semispherical protrusions in a garment for providing therapy against psychiatric conditions by providing tactile stimulation to the body of the user. Accordingly, a need persists for a pressure stimulation garment which on the one hand is capable of providing a tactile sensory stimulation to the skin of the wearer and which on the other hand does not provide the same degree of stigmatization of the user solely due to the curvy outer surface of that garment.

It is an objective of the present invention to provide technologies fulfilling this need.

### Brief description of the invention

This objective is fulfilled according to the present invention in its various aspects.

Accordingly, the present invention relates in a first aspect to a garment for providing tactile sensory stimulation to the torso of a user, wherein said garment comprises:
- a first front portion;
- a second front portion;
- a back portion;

wherein the first front portion is being connected to the back portion in such a way that a first throughgoing opening for a right arm is being present in the garment;
wherein the second front portion is being connected to the back portion in such a way that a second throughgoing opening for a left arm is being present in the garment;
wherein the first front portion comprises an inner lining of a fabric layer and an outer fabric layer;
wherein the second front portion comprises an inner lining of a fabric layer and an outer fabric layer;
wherein the back portion comprises an inner lining of a fabric layer and an outer fabric layer; wherein in respect of the first front portion, the second front portion and the back portion, said portion comprises a plurality of stimulation elements;
characterized in that
in respect of one or more of said stimulation elements, preferably in respect of all said stimulation elements, said stimulation element comprises a first surface and an opposite second surface separated by a rim portion, wherein said first surface is defining an essentially flat surface and wherein said second surface is having a convex shape;
wherein in respect of one or more of said stimulation elements, preferably in respect of all said stimulation elements, said stimulation element is being arranged between said inner lining of fabric layer and said outer fabric layer in such a way that said first surface of said stimulation element faces said outer fabric layer, and in such a way that said second surface of said stimulation element faces said inner lining of fabric layer;
wherein in respect of said one or more stimulation elements, preferably in respect of each said stimulation elements, said first surface of said stimulation element independently is having an area of 2 - 25 cm² and/or
wherein in respect of said one or more stimulation elements, preferably in respect of each said stimulation elements, the distance between said essentially flat surface defined by said first surface and the most distant point (P) of said second surface independently is selected from the ranges of 1.0 - 5.5 cm.

The present invention provides a garment having stimulation elements incorporated therein in a special way so that the presence of the stimulation elements is not easily recognizable by other persons in that the outer surface of the garment to a larger extent will have a smooth surface.

Hereby, less risk of stigmatizing behavior from other persons is achieved.

### Brief description of the figures

Fig. 1 is a plan view illustrating a garment according to the first aspect of the present invention as seen from its front.
Fig. 2 is a plan view illustrating a garment of Fig. 1 as seen from its back.
Fig. 3a - 3f are cross-sectional views of various shapes of stimulation elements to be used with the garment according to the present invention.
Fig. 4 is a cross-sectional view illustrating how the stimulation elements are being accommodated within confined sectors made in the fabric of the garment.
Fig. 5 is a diagram illustrating use of stitches of threads to form confined sectors for accommodating the stimulation elements in the fabric of the garment.

### Detailed description of the invention

### The first aspect of the present invention

In a first aspect the present invention relates to a garment 100 for providing tactile sensory stimulation to the torso of a user, wherein said garment comprises:
- a first front portion 2;
- a second front portion 4;
- a back portion 6;

wherein the first front portion 2 is being connected to the back portion 6 in such a way that a first throughgoing opening 8 for a right arm is being present in the garment;
wherein the second front portion 4 is being connected to the back portion 6 in such a way that a second throughgoing opening 10 for a left arm is being present in the garment;
wherein the first front portion 2 comprises an inner lining 12 of a fabric layer and an outer fabric layer 14;
wherein the second front portion 4 comprises an inner lining 16 of a fabric layer and an outer fabric layer 18;
wherein the back portion 6 comprises an inner lining 20 of a fabric layer and an outer fabric layer 22;
wherein in respect of the first front portion 2, the second front portion 4 and the back portion 6, said portion comprises a plurality of stimulation elements 24;
characterized in that
in respect of one or more of said stimulation elements 24, preferably in respect of all said stimulation elements 24, said stimulation element comprises a first surface 26 and an opposite second surface 28 separated by a rim portion 30, wherein said first surface 26 is defining an essentially flat surface 32 and wherein said second surface 22 is having a convex shape;
wherein in respect of one or more of said stimulation elements 24, preferably in respect of all said stimulation elements, said stimulation element 24 is being arranged between said inner lining 12,16,20 of fabric layer and said outer fabric layer 14,18,22 in such a way that said first surface 26 of said stimulation element 24 faces said outer fabric layer 14,18,22, and in such a way that said second surface of said stimulation element faces said inner lining 12,16,20 of fabric layer;
wherein in respect of said one or more stimulation elements 24, preferably in respect of each said stimulation elements, said first surface 26 of said stimulation element independently is having an area of 2 - 25 cm² and/or
wherein in respect of said one or more stimulation elements 24, preferably in respect of each said stimulation elements, the distance between said essentially flat surface 32 defined by said first surface 26 and the most distant point P of said second surface 28 independently is selected from the ranges of 1.0 - 5.5 cm.

Accordingly, the garment of the first aspect of the present invention comprises an inner lining and an outer fabric layer. Between these layers a plurality of stimulations element are arranged. The stimulation elements comprises a first surface and an opposite second surface, wherein said first surface is defining an essentially flat surface and wherein said second surface is having a convex shape. The stimulation elements are arranged between the inner lining and the outer fabric layer in such a way that the first surface faces the outer fabric layer.

Hereby, when viewed by other persons from the outside, the garment will have a rather smooth outer surface. This appearance will aid in minimizing any stigmatizing behavior from other people.

In an embodiment of the garment of the first aspect of the present invention, the garment is being in the form of a vest.

This embodiment represents a very simple form of a garment which is easy to wear and which yet provides the desired effect.

In an embodiment of the garment of the first aspect of the present invention, the garment additionally comprises a right sleeve which is being connected at said first throughgoing opening 8 of said garment; and a left sleeve which is being connected at said second throughgoing opening 10 of said garment, wherein said garment 6 is in the form of a jacket.

In an embodiment of this embodiment, the right sleeve and/or the left sleeve of the garment comprises an inner lining of a fabric layer and an outer fabric layer;
wherein in respect of the right sleeve and/or the left sleeve, said sleeve(s) comprise(s) a plurality of stimulation elements 24;
wherein in respect of one or more of said stimulation elements 24 of said sleeve(s), preferably in respect of all said stimulation elements 24 of said sleeve(s), said stimulation element comprises a first surface 26 and an opposite second surface 28 separated by a rim portion 30, wherein said first surface 26 is defining an essentially flat surface 32 and wherein said second surface 22 is having a convex shape;
wherein in respect of one or more of said stimulation elements 24, preferably in respect of all said stimulation elements, said stimulation element 24 is being arranged between said inner lining of fabric layer of said sleeve and said outer fabric layer of said sleeve in such a way that said first surface 26 of said stimulation element 24 faces said outer fabric layer of said sleeve, and in such a way that said second surface of said stimulation element faces said inner lining of fabric layer of said sleeve.

A jacket is not as simple a garment as a vest. However, a jacket enables stimulation elements to provide stimulation to the arms of a user.

In an embodiment of the garment of the first aspect of the present invention and in respect of said one or more stimulation elements 24, preferably in respect of each said stimulation elements, said stimulation element 24 is being attached to said outer fabric layer 14,18,22 by attachment by an adhesive at said first surface 26 of said stimulation element 24.

Hereby an easy way of attachment of the stimulation elements to the outer fabric layer is attained.

In an embodiment of the garment of the first aspect of the present invention and in respect of said one or more stimulation elements 24, preferably in respect of each said stimulation elements, said stimulation element 24 is being attached to said outer fabric layer 14,18,22 by attachment by hook and loop parts of a hook-and-loop-part fastener.

In an embodiment of this embodiment the first surface 26 of said stimulation element 24 comprises a hook part of a hook-and-loop-part fastener, and said outer fabric layer 14,18,22, at the position of said stimulation element 24 comprises a loop part of a hook-and-loop-part fastener; or said first surface 26 of said stimulation element 24 comprises a loop part of a hook-and-loop-part fastener, and said outer fabric layer 14,18,22, at the position of said stimulation element 24 comprises a hook part of a hook-and-loop-part fastener.

Hook-and-loop-part fasteners provides an alternative and easy way of attachment of the stimulation elements to the outer fabric layer. Hook-and-loop fasteners are well-known under the tradename Vecro^{®}.

In an embodiment of the garment of the first aspect of the present invention and in respect of a plurality of said stimulation elements 24, preferably in respect of all said stimulation elements, said stimulation element 24 is being arranged inside a tubing of fabric and said tubing of fabric is being fastened to said outer fabric layer 14,18,22.

This is yet an alternative and easy way of attachment of the stimulation elements to the outer fabric layer.

In an embodiment of this embodiment, the tubing of fabric, between positions of stimulation elements 24, comprises stitches of a thread, thereby forming individual cells in said tubing of fabric, wherein or more of said stimulation elements is/are being confined in such cells.

Hereby an adequate distance between the stimulation elements can be attained.

In an embodiment of the garment of the first aspect of the present invention and in respect of said first front portion 2, said second front portion 4 and/or said back portion 6, said inner lining 12,16,20 of a fabric layer and said outer fabric layer 14,18,22 thereof are forming a plurality of confined sectors 34 which are being delimited in relation to each other by stitches 36 of thread which connects said inner lining 12,16,18 of a fabric layer and said outer fabric layer 14,18,22 and wherein in respect of one or more of said stimulation elements 24 of said part of the garment, preferably all said stimulation elements of said part of the garment, said stimulation element(s) 24 is/are being arranged within such a confined sector 34.

Hereby, the stimulation elements can be held in place in a very simple way.

In an embodiment of the garment of the first aspect of the present invention and in respect of one or more confined sectors 34 of said first front portion 2, said second front portion 4 and/or said back portion 6 of said garment, said interior of said confined sector 34 is having a base area A, as measured as the area within said stitches 36 surrounding said confined sector, is selected from the ranges of 6 - 100 cm², such as 10 - 95 cm², such as 15 - 90 cm², e.g. 20 - 85 cm², for example 25 - 80 cm², such as 30 - 75 cm², such as 35 - 70 cm², for example 40 - 65 cm², e.g. 45 - 60 cm² or 50 - 55 cm².

These magnitudes of areas are suitable for a garment for use by a child or by an adult.

In an embodiment of the garment of the first aspect of the present invention and in respect of one or more confined sectors 34 of said first front portion 2, said second front portion 4 and/or back portion 6, the base area A of said interior of said confined sector is having the shape of a triangle, a tetragon, such as a rectangle, a square, a rhombus or a hexagon.

These shapes are suitable for the intended purpose and allows for creating a repeating pattern (tessellation) of similar shapes of confined sectors.

In an embodiment of the garment of the first aspect of the present invention one or more of said confined sectors 34 independently is/are comprising one, two, three, four or more of said stimulation elements 24.

These numbers of stimulation elements in each confined sector have proven suitable.

In an embodiment of the garment of the first aspect of the present invention and in respect of one or more of said confined sectors 34, preferably in respect of all said confined sectors, and in respect of one or more of said stimulation elements 24, preferably in respect of all stimulation elements 24, the dimension and geometry of said confined sector 34 has been adapted to the dimension and geometry of said stimulation elements 24 accommodated therein in such a way, that said stimulation element(s) will not be able to change its/their orientation so that its/their first surface start(s) facing the inner lining 12,16,20 of said garment.

In an embodiment of the garment of the first aspect of the present invention and in respect of said one or more stimulation elements 24, preferably in respect of each said stimulation elements, said stimulation element 24 independently is being made from a polymer, such as rubber, silicone or plastic; wood; metal or an alloy, a ceramic material, such as glass, or a foamed material.

These materials are suitable for providing the desired tactile stimulation effect to a user wearing the garment.

In an embodiment of the garment of the first aspect of the present invention and in respect of said one or more of said stimulation elements 24, preferably in respect of each said stimulation elements, said stimulation element 24 independently is having a weight of 10 - 150 g, such as 15 - 140 g, for example 20 - 135 g, for example, 25 - 120 g, such as 30 - 115 g, such as 35 - 110 g, e.g. 40 - 105 g, such as 45 - 100 g, for example 50 - 95 g, such as 55 - 90 g, e.g. 60 - 85 g, such as 65 - 80 g or 70 - 75 g.

These magnitudes of weights of the stimulation elements are suitable for providing the desired tactile stimulation effect to a user wearing the garment.

In an embodiment of the garment of the first aspect of the present invention and in respect of said one or more stimulation elements 24, preferably in respect of each said stimulation elements, said first surface 26 of said stimulation element independently is having an area of 3 - 24 cm², for example 4 - 23 cm², e.g. 5 - 22 cm², such as 6 - 21 cm², for example 7 - 20 cm², for example 8 - 19 cm², such as 9 - 18 cm², e.g. 10 - 17 cm², such as 11 - 18 cm², for example 12 - 17 cm², such as 13 - 16 cm² or 14 - 15 cm².

These magnitudes of areas are suitable for concealing the presence of the stimulation elements.

In an embodiment of the garment of the first aspect of the present invention and in respect of said one or more stimulation elements 24, preferably in respect of each said stimulation elements, the distance between said essentially flat surface 32 defined by said first surface 26 and the most distant point P of said second surface 28 independently is selected from the ranges of 1.5 - 5.0 cm, for example 2.0 - 4.5 cm, such as 2.5 - 4.0 cm or 3.0 - 3.5 cm.

These magnitudes of distances are suitable for providing the desired tactile stimulation effect to a user wearing the garment and also for concealing the presence of the stimulation elements.

In an embodiment of the garment of the first aspect of the present invention the number of stimulation elements 24 being present at said first front portion 2 is being selected from the ranges of 5 - 40, such as 10 - 35, e.g. 15 - 30, such as 20 - 25; and/or wherein the number of stimulation elements 24 being present at said second front portion 4 is being selected from the ranges of 5 - 40, such as 10 - 35, e.g. 15 - 30, such as 20 - 25; and/or wherein the number of stimulation elements 24 being present at said back portion 6 is being selected from the ranges of 10 - 85, such as 15 - 80, e.g. 20 - 75, such as 25 - 70, for example 30 - 65, such as 35 - 60, for example 40 - 55 or 45 - 50.

These numbers of stimulation elements have proven suitable for providing the desired tactile stimulation effect to a user wearing the garment.

In an embodiment of the garment of the first aspect of the present invention and in respect of said one or more stimulation elements 24, preferably in respect of each said stimulation elements, the stimulation element 24 independently is having the shape of a cut-through sphere, a cut-through ellipsoid, a cut-through ovoid, a cone, a cone with rounded apex, a tetrahedron, a tetrahedron with rounded apex, a prism, a prism with a rounded edge.

These geometries will represent a shape which is suitable for providing the desired tactile stimulation effect to a user wearing the garment.

In an embodiment of the garment of the first aspect of the present invention and in respect of said one or more stimulation elements 24, preferably in respect of each said stimulation elements, the first surface 26 of said stimulation element 24 is being an essentially flat surface.

This geometry is a simple embodiment of the stimulation element which is easy and cost efficient to produce.

In an embodiment of the garment of the first aspect of the present invention and in respect of one or more of said stimulation elements 24, said first surface 26 of said stimulation element 24 is comprising a cavity 46.

A cavity may be selected to lower the weight of the stimulation element.

In an embodiment of the garment of the first aspect of the present invention and in respect of said first front portion 2, said second front portion 4 and/or said back portion 6 of said garment, said garment additionally comprises a padding layer which is being arranged between an inner surface of the outer fabric layer 14,18,22 and said essentially flat surface 32 of said first surface 26 of said stimulation elements 24, such as at an outer surface of said inner lining 12,16,20 of a fabric layer.

Hereby improved thermal insulation may be attained.

In an embodiment of the garment of the first aspect of the present invention and in respect of said first front portion 2, and/or said second front portion 4, said portion additionally comprises one or more pockets 44.

In an embodiment of the garment of the first aspect of the present invention, said garment comprises a zipper 42 arranged along an edge 38 of the first front portion 2 and along an edge 30 of the second front portion 4 for zipping/unzipping said first front portion 2 to/from said second front portion 4.

In an embodiment of the garment of the first aspect of the present invention said garment comprises one or more buttons and corresponding one or more buttonholes, wherein said one or more buttons are being arranged along an edge 38 of the first front portion 2 of said garment and wherein said one or more buttonholes are being arranged along an edge 40 of the second front portion 4 of said garment; or wherein said one or more buttonholes are being arranged along an edge 38 of the first front portion 2 of said garment and wherein said one or more buttons are being arranged along an edge 40 of the second front portion 4 of said garment.

Zippers and buttons allows for closing the opening formed by the first front portion and the second front portion.

In an embodiment of the garment of the first aspect of the present invention said garment comprises one or more, such as two, three or four cords, wherein in respect of each cord, said first front portion 2 comprises a first opening in its inner lining 12 proximate to the edge 38 of the first front portion 2, and said second front portion 4 comprises a second opening in its inner lining 16 proximate to the edge 40 of the second front portion 4, wherein said cord extends between said first opening and said second opening between the inner lining 12,16,20 of the garment and the outer fabric layer 14,18,22 of the garment, and in such a way that the first end of said cord exits the first opening in the inner lining 14 of the first front portion 2 of the garment, and in such a way that the second end of said cord exits the second opening in the inner lining 16 of the second front portion 4 of the garment, thereby allowing tensioning the garment around the torso of the user.

In an embodiment of the garment of the present invention, the garment is for use in therapy against psychiatric conditions; wherein the psychiatric condition optionally is selected from the group of: Attention Deficit Hyperactivity Disorder (ADHD), Sensory Processing Disorder (SPD), disorders belonging to the autism spectrum, depression, anxiety, restlessness, dementia, cognitive disorders.

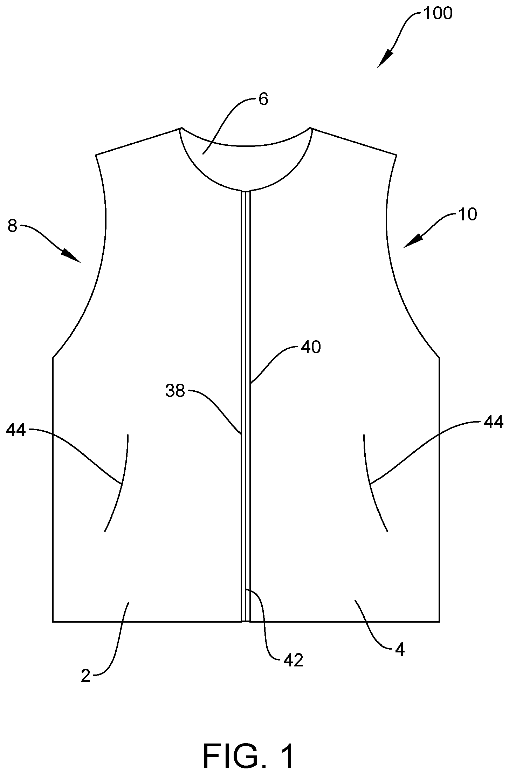

In order to better explain the present invention in its various aspect we now refer to the drawings, wherein Fig. 1 is a plan view illustrating a garment according to the first aspect of the present invention as seen from its front.

Fig. 1 shows that the garment 100 comprises a first front portion 2, a second front portion 4 and a back portion 6.

The first front portion 2 is being connected to the back portion 6 in such a way that a first throughgoing opening 8 for a right arm is being present in the garment, and the second front portion 4 is being connected to the back portion 6 in such a way that a second throughgoing opening 10 for a left arm is being present in the garment. The garment illustrated in Fig. 1 is in the form of a vest to be worn by a user in need thereof.

A zipper 42 is arranged along an edge 38 of the first front portion 2 and along an edge 30 of the second front portion 4 for zipping/unzipping said first front portion 2 to/from said second front portion 4.

It is seen that the first front portion 2 and the second front portion 4 have been provided with a pocket 44.

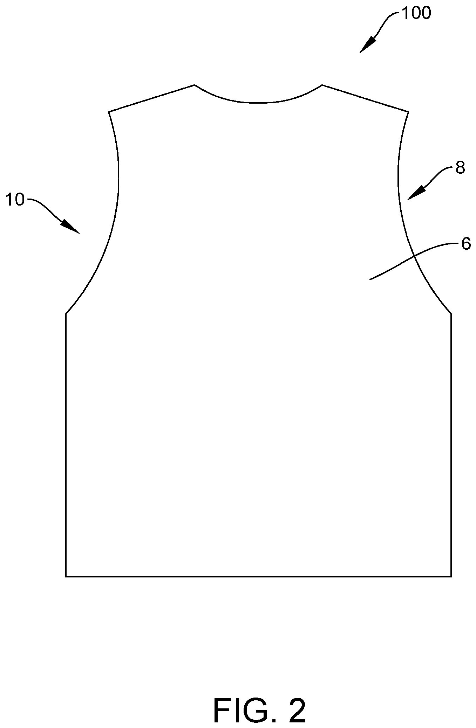

Fig. 2 is a plan view illustrating the garment of Fig. 1 as seen from its back.

The first front portion 2 of the garment comprises an inner lining 12 of a fabric layer and an outer fabric layer 14, and the second front portion 4 comprises an inner lining 16 of a fabric layer and an outer fabric layer 18 (not seen in Fig. 1 or 2). Moreover, the back portion 6 comprises an inner lining 20 of a fabric layer and an outer fabric layer 22 (not seen in Fig. 1 or 2).

Hereby it is possible to arrange a plurality of stimulation elements in various portions of the garment and hold these in place between the inner lining of a fabric layer 12,16,20 and the outer fabric layer 14,18,22 as explained below.

The inclusion of the stimulation elements in the garment will accordingly provide tactile sensory stimulation to the torso of a user wearing the garment.

The stimulation elements themselves are having a special design that to a certain extension conceal the presence thereof between two layers of the garment.

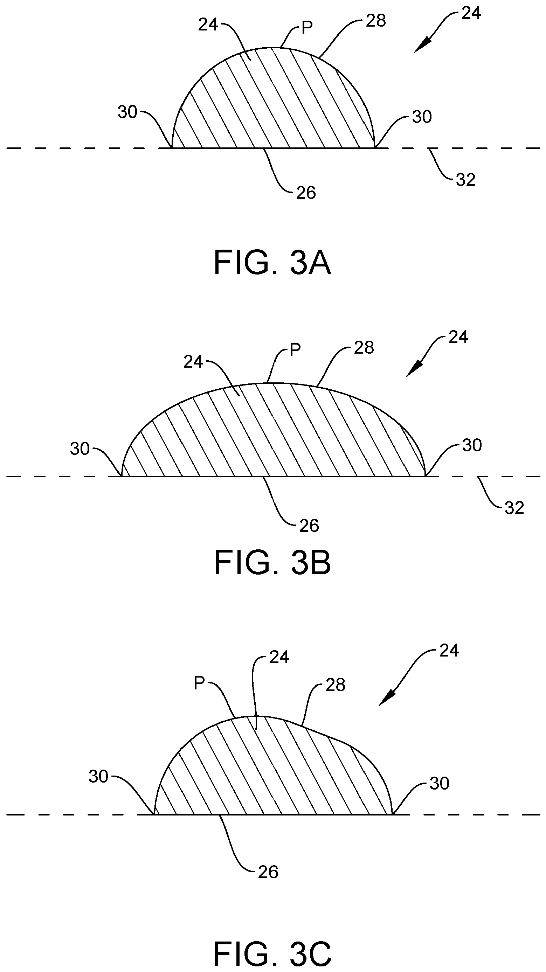

This is further illustrated in Fig. 3a - 3f.

Fig. 3a - 3f are cross-sectional views of various shapes of stimulation elements to be used with the garment according to the present invention.

Fig. 3a shows the stimulation element 24 in the shape of a cut-through sphere. Fig. 3a shows that the stimulation element comprises a first surface 26 and an opposite second surface 28 separated by the rim portion 30 therebetween. It is seen that the first surface 26 is defining an essentially flat surface 32 and that the second surface 22 is having a convex shape of a spherical surface.

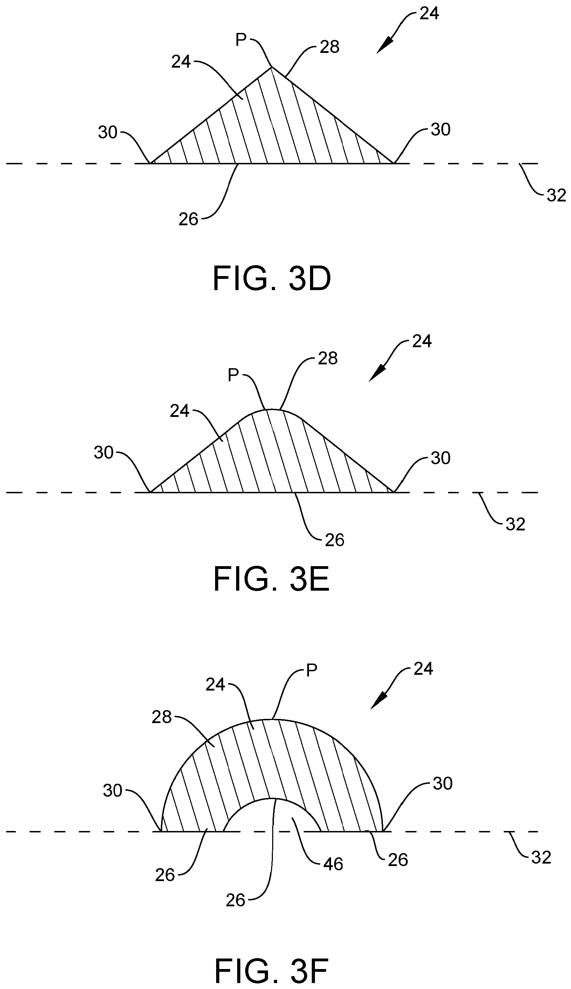

Fig. 3b, 3c, 3d and 3e illustrated corresponding features of stimulation elements having the shape of a cut-through ellipsoid, a cut-through ovoid, a cone and a cone with rounded apex, respectively.

Fig. 3f is a cross-sectional view of an embodiment of a stimulation element 24, wherein the first surface is partly concave, yet still defines an essentially flat surface 32 in respect of the remainder of the first surface 26.

The stimulation elements 24 are being arranged between the inner lining 12,16,20 of fabric layer and the outer fabric layer 14,18,22 in such a way that the first surface 26 of the stimulation element 24 faces the outer fabric layer 14,18,22, and in such a way that the second surface of the stimulation element faces the inner lining 12,16,20 of fabric layer.

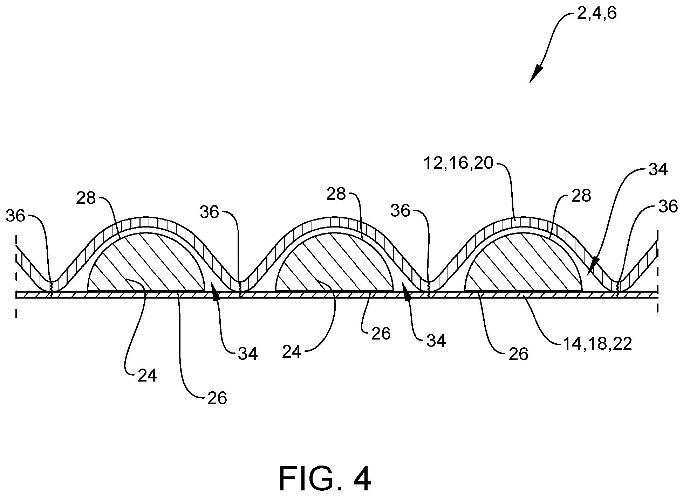

This is illustrated in Fig. 4.

Fig. 4 is a cross-sectional view illustrating how the stimulation elements are being accommodated within confined sectors made in the fabric of the garment.

Fig. 4 shows that in respect of the first front portion 2, the second front portion 4 and the back portion 6, the inner lining 12,16,20 of a fabric layer and the outer fabric layer 14,18,22 thereof are forming a plurality of confined sectors 34 which are being delimited in relation to each other by stitches 36 of thread. The thread connects the inner lining 12,16,18 of a fabric layer with the outer fabric layer 14,18,22.

It is seen that stimulation elements 24 are being arranged within the formed confined sectors 34 in a way so that the first surface 26 of the stimulation elements 24 faces the outer fabric layer 14,18,22, and in way so that the second surface 28 of the stimulation elements 24 faces the inner lining 12,16,20 of fabric layer.

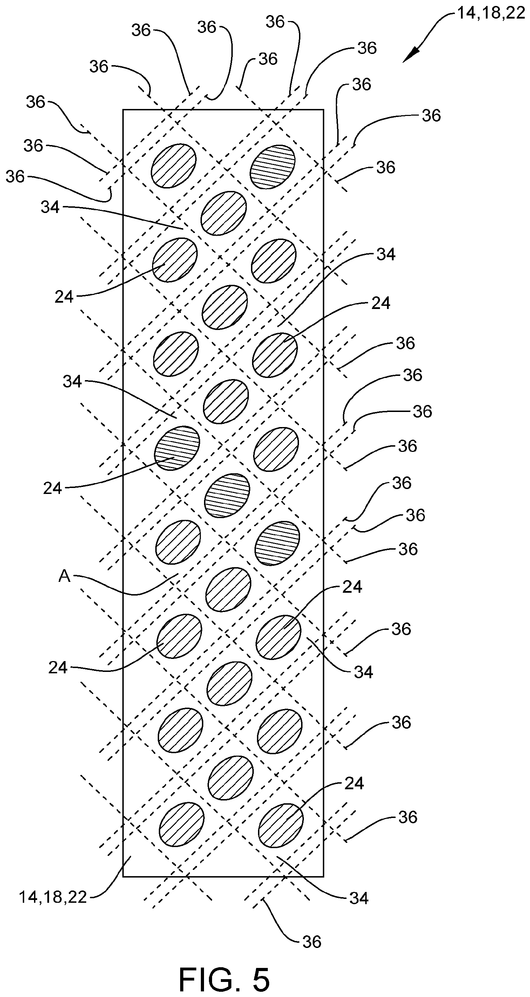

Fig. 5 is a diagram illustrating use of stitches of threads to form confined sectors for accommodating the stimulation elements in the fabric of the garment.

Fig. 5 shows the outer fabric layer 14,18,22 of a first front portion, a second front portion or a back portion of a garment, respectively. A plurality of stitches 36 of threads have been sewed so as to connect the outer fabric layer 14,18,22 with the corresponding inner lining of fabric layer 12,16,20 (not seen in Fig. 4).

The stitches 36 are either parallel or perpendicular to each other. Hereby a plurality of confined sectors 34 are formed which accommodate a stimulation element 24.

Fig. 5 also illustrates the concept of defining a magnitude of a base area A of a confined sector. The base area A is defined as the area confined withing the stitches as shown.

### List of reference numerals

- 2: First front portion of garment
- 4: Second front portion of garment
- 6: Back portion of garment
- 8: First through-going opening for a first sleeve
- 10: Second through-going opening for a first sleeve
- 12: Inner lining of fabric layer of first front portion
- 14: Outer fabric layer of first front portion
- 16: Inner lining of fabric layer of second front portion
- 18: Outer fabric layer of first front portion
- 20: Inner lining of fabric layer of back portion
- 22: Outer fabric layer of back portion
- 24: Stimulation element
- 26: First surface of stimulation element
- 28: Second surface of stimulation element
- 30: Rim portion between first and second surface of stimulation element
- 32: Flat surface defined by first surface of stimulation element
- 34: Confined sector between inner lining of fabric layer and outer fabric layer
- 36: Stitch of tread
- 38: Edge of first front portion of garment
- 40: Edge of second front portion of garment
- 42: Zipper
- 44: Pocket
- 46: Cavity of first surface of stimulation element
- 100: Garment
- A: Base area of confined sector
- P: Most distance point on second surface of stimulation element from first surface

## Claims

1. A garment (100) for providing tactile sensory stimulation to the torso of a user, wherein said garment comprises:
- a first front portion (2);
- a second front portion (4);
- a back portion (6);
wherein the first front portion (2) is being connected to the back portion (6) in such a way that a first throughgoing opening (8) for a right arm is being present in the garment;
wherein the second front portion (4) is being connected to the back portion (6) in such a way that a second throughgoing opening (10) for a left arm is being present in the garment;
wherein the first front portion (2) comprises an inner lining (12) of a fabric layer and an outer fabric layer (14);
wherein the second front portion (4) comprises an inner lining (16) of a fabric layer and an outer fabric layer (18);
wherein the back portion (6) comprises an inner lining (20) of a fabric layer and an outer fabric layer (22);
wherein in respect of the first front portion (2), the second front portion (4) and the back portion (6), said portion comprises a plurality of stimulation elements (24);
**characterized in that**
in respect of one or more of said stimulation elements (24), preferably in respect of all said stimulation elements (24), said stimulation element comprises a first surface (26) and an opposite second surface (28) separated by rim portion (30), wherein said first surface (26) is defining an essentially flat surface (32) and wherein said second surface (22) is having a convex shape;
wherein in respect of one or more of said stimulation elements (24), preferably in respect of all said stimulation elements, said stimulation element (24) is being arranged between said inner lining (12,16,20) of fabric layer and said outer fabric layer (14,18,22) in such a way that said first surface (26) of said stimulation element (24) faces said outer fabric layer (14,18,22), and in such a way that said second surface of said stimulation element faces said inner lining (12,16,20) of fabric layer;
wherein in respect of said one or more stimulation elements (24), preferably in respect of each said stimulation elements, said first surface (26) of said stimulation element independently is having an area of 2 - 25 cm² and/or
wherein in respect of said one or more stimulation elements (24), preferably in respect of each said stimulation elements, the distance between said essentially flat surface (32) defined by said first surface (26) and the most distant point (P) of said second surface (28) independently is selected from the ranges of 1.0 - 5.5 cm.

2. A garment (100) according to claim 1, wherein said garment is being in the form of a vest.

3. A garment (100) according to claim 1, wherein said garment additionally comprises a right sleeve which is being connected at said first throughgoing opening (8) of said garment; and a left sleeve which is being connected at said second throughgoing opening (10) of said garment, wherein said garment (6) is in the form of a jacket.

4. A garment (100) according to claim 3, wherein said right sleeve and/or said left sleeve of said garment comprises an inner lining of a fabric layer and an outer fabric layer;
wherein in respect of the right sleeve and/or the left sleeve, said sleeve(s) comprise(s) a plurality of stimulation elements (24);
wherein in respect of one or more of said stimulation elements (24) of said sleeve(s), preferably in respect of all said stimulation elements (24) of said sleeve(s), said stimulation element comprises a first surface (26) and an opposite second surface (28) separated by rim portion (30), wherein said first surface (26) is defining an essentially flat surface (32) and wherein said second surface (22) is having a convex shape;
wherein in respect of one or more of said stimulation elements (24), preferably in respect of all said stimulation elements, said stimulation element (24) is being arranged between said inner lining of fabric layer of said sleeve and said outer fabric layer of said sleeve in such a way that said first surface (26) of said stimulation element (24) faces said outer fabric layer of said sleeve, and in such a way that said second surface of said stimulation element faces said inner lining of fabric layer of said sleeve.

5. A garment (100) according to any of the preceding claims, wherein in respect of said one or more stimulation elements (24), preferably in respect of each said stimulation elements, said stimulation element (24) is being attached to said outer fabric layer (14,18,22) by attachment by an adhesive at said first surface (26) of said stimulation element (24);
and/or
wherein in respect of said one or more stimulation elements (24), preferably in respect of each said stimulation elements, said stimulation element (24) is being attached to said outer fabric layer (14,18,22) by attachment by hook and loop parts of a hook-and-loop-part fastener;
optionally wherein said first surface (26) of said stimulation element (24) comprises a hook part of a hook-and-loop-part fastener, and wherein said outer fabric layer (14,18,22), at the position of said stimulation element (24) comprises a loop part of a hook-and-loop-part fastener; or optionally wherein said first surface (26) of said stimulation element (24) comprises a loop part of a hook-and-loop-part fastener, and wherein said outer fabric layer (14,18,22), at the position of said stimulation element (24) comprises a hook part of a hook-and-loop-part fastener.

6. A garment (100) according to any of the preceding claims, wherein in respect of a plurality of said stimulation elements (24), preferably in respect of all said stimulation elements, said stimulation element (24) is being arranged inside a tubing of fabric and wherein said tubing of fabric is being fastened to said outer fabric layer (14,18,22); optionally wherein said tubing of fabric, between positions of stimulation elements (24) comprises stitches of a thread, thereby forming individual cells in said tubing of fabric, wherein or more of said stimulation elements is/are being confined in such cells.

7. A garment (100) according to any of the preceding claims, wherein in respect of said first front portion (2) said second front portion (4) and/or said back portion (6), said inner lining (12,16,20) of a fabric layer and said outer fabric layer (14,18,22) thereof are forming a plurality of confined sectors (34) which are being delimited in relation to each other by stitches (36) of thread which connects said inner lining (12,16,18) of a fabric layer and said outer fabric layer (14,18,22) and wherein in respect of one or more of said stimulation elements (24) of said part of the garment, preferably all said stimulation elements of said part of the garment, said stimulation element(s) (24) is/are being arranged within such a confined sector (34);
optionally wherein the in respect of one or more confined sectors (34) of said first front portion (2), said second front portion (4) and/or said back portion (6) of said garment, said interior of said confined sector (34) is having a base area (A), as measured as the area within said stitches (36) surrounding said confined sector, is selected from the ranges of 6 - 100 cm², such as 10 - 95 cm², such as 15 - 90 cm², e.g. 20 - 85 cm², for example 25 - 80 cm², such as 30 - 75 cm², such as 35 - 70 cm², for example 40 - 65 cm², e.g. 45 - 60 cm² or 50 - 55 cm²;
and/or
wherein in respect of one or more confined sectors (34) of said first front portion (2), said second front portion (4) and/or back portion (6), the base area (A) of said interior of said confined sector is having the shape of a triangle, a tetragon, such as a rectangle, a square, a rhombus or a hexagon;
and/or
wherein one or more of said confined sectors (34) independently is/are comprising one, two, three, four or more of said stimulation elements (24);
and/or
wherein in respect of one or more of said confined sectors (34), preferably in respect of all said confined sectors, and in respect of one or more of said stimulation elements (24), preferably in respect of all stimulation elements (24), the dimension and geometry of said confined sector (34) has been adapted to the dimension and geometry of said stimulation elements (24) accommodated therein in such a way, that said stimulation element(s) will not be able to change its/their orientation so that its/their first surface start(s) facing the inner lining (12,16,20) of said garment.

8. A garment (100) according to any of the preceding claims, wherein in respect of said one or more stimulation elements (24), preferably in respect of each said stimulation elements, said stimulation element (24) independently is being made from a polymer, such as rubber, silicone or plastic; wood; metal or an alloy, a ceramic material, such as glass, or a foamed material.

9. A garment (100) according to any of the preceding claims, wherein in respect of said one or more of said stimulation elements (24), preferably in respect of each said stimulation elements, said stimulation element (24) independently is having a weight of 10 - 150 g, such as 15 - 140 g, for example 20 - 135 g, for example, 25 - 120 g, such as 30 - 115 g, such as 35 - 110 g, e.g. 40 - 105 g, such as 45 - 100 g, for example 50 - 95 g, such as 55 - 90 g, e.g. 60 - 85 g, such as 65 - 80 g or 70 - 75 g;
and/or
wherein in respect of said one or more stimulation elements (24), preferably in respect of each said stimulation elements, said first surface (26) of said stimulation element independently is having an area of 3 - 24 cm², for example 4 - 23 cm², e.g. 5 - 22 cm², such as 6 - 21 cm², for example 7 - 20 cm², for example 8 - 19 cm², such as 9 - 18 cm², e.g. 10 - 17 cm², such as 11 - 18 cm², for example 12 - 17 cm², such as 13 - 16 cm² or 14 - 15 cm²;
and/or
wherein in respect of said one or more stimulation elements (24), preferably in respect of each said stimulation elements, the distance between said essentially flat surface (32) defined by said first surface (26) and the most distant point (P) of said second surface (28) independently is selected from the ranges of 1.5 - 5.0 cm, for example 2.0 - 4.5 cm, such as 2.5 - 4.0 cm or 3.0 - 3.5 cm.

10. A garment (100) according to any of the preceding claims, wherein the number of stimulation elements (24) being present at said first front portion (2) is being selected from the ranges of 5 - 40, such as 10 - 35, e.g. 15 - 30, such as 20 - 25; and/or wherein the number of stimulation elements (24) being present at said second front portion (4) is being selected from the ranges of 5 - 40, such as 10 - 35, e.g. 15 - 30, such as 20 - 25; and/or wherein the number of stimulation elements (24) being present at said back portion (6) is being selected from the ranges of 10 - 85, such as 15 - 80, e.g. 20 - 75, such as 25 - 70, for example 30 - 65, such as 35 - 60, for example 40 - 55 or 45 - 50.

11. A garment (100) according to any of the preceding claims, wherein in respect of said one or more stimulation elements (24), preferably in respect of each said stimulation elements, the stimulation element (24) independently is having the shape of a cut-through sphere, a cut-through ellipsoid, a cut-through ovoid, a cone, a cone with rounded apex, a tetrahedron, a tetrahedron with rounded apex, a prism, a prism with a rounded edge;
and/or
wherein in respect of said one or more stimulation elements (24), preferably in respect of each said stimulation elements, the first surface (26) of said stimulation element (24) is being an essentially flat surface;
and/or
wherein in respect of one or more of said stimulation elements (24), said first surface (26) of said stimulation element (24) is comprising a cavity (46).

12. A garment (100) according to any of the preceding claims, wherein in respect of said first front portion (2), said second front portion (4) and/or said back portion (6) of said garment, said garment additionally comprises a padding layer which is being arranged between an inner surface of the outer fabric layer (14,18,22) and said essentially flat surface (32) of said first surface (26) of said stimulation elements (24), such as at an outer surface of said inner lining (12,16,20) of a fabric layer.

13. A garment (100) according to any of the preceding claims, wherein in respect of said first front portion (2), and/or said second front portion (4), said portion additionally comprises one or more pockets (44).

14. A garment (100) according to any of the preceding claims, wherein said garment comprises a zipper (42) arranged along an edge (38) of the first front portion (2) and along an edge (30) of the second front portion (4) for zipping/unzipping said first front portion (2) to/from said second front portion (4); or
wherein said garment comprises one or more buttons and corresponding one or more buttonholes, wherein said one or more buttons are being arranged along an edge (38) of the first front portion (2) of said garment and wherein said one or more buttonholes are being arranged along an edge (40) of the second front portion (4) of said garment; or wherein said one or more buttonholes are being arranged along an edge (38) of the first front portion (2) of said garment and wherein said one or more buttons are being arranged along an edge (40) of the second front portion (4) of said garment.

15. A garment (100) according to any of the claims 1 - 14 for use in therapy against psychiatric conditions; wherein the psychiatric condition optionally is selected from the group of: Attention Deficit Hyperactivity Disorder (ADHD), Sensory Processing Disorder (SPD), disorders belonging to the autism spectrum, depression, anxiety, restlessness, dementia, cognitive disorders.

## Patentansprüche

1. Kleidungsstück (100) zum Bereitstellen einer taktilen sensorischen Stimulation für den Rumpf eines Benutzers, wobei das Kleidungsstück Folgendes umfasst:
- einen ersten vorderen Abschnitt (2);
- einen zweiten vorderen Abschnitt (4);
- einen hinteren Abschnitt (6);
wobei der erste vordere Abschnitt (2) derart mit dem hinteren Abschnitt (6) verbunden ist, dass eine erste durchgehende Öffnung (8) für einen rechten Arm in dem Kleidungsstück vorhanden ist;
wobei der zweite vordere Abschnitt (4) derart mit dem hinteren Abschnitt (6) verbunden ist, dass eine zweite durchgehende Öffnung (10) für einen linken Arm in dem Kleidungsstück vorhanden ist;
wobei der erste vordere Abschnitt (2) eine innere Auskleidung (12) aus einer Gewebelage und eine äußere Gewebelage (14) umfasst;
wobei der zweite vordere Abschnitt (4) eine innere Auskleidung (16) aus einer Gewebelage und eine äußere Gewebelage (18) umfasst;
wobei der hintere Abschnitt (6) eine innere Auskleidung (20) aus einer Gewebelage und eine äußere Gewebelage (22) umfasst;
wobei in Bezug auf den ersten vorderen Abschnitt (2), den zweiten vorderen Abschnitt (4) und den hinteren Abschnitt (6) der Abschnitt eine Vielzahl von Stimulationselementen (24) umfasst;
**dadurch gekennzeichnet, dass**
in Bezug auf eines oder mehrere der Stimulationselemente (24), bevorzugt in Bezug auf alle Stimulationselemente (24), das Stimulationselement eine erste Oberfläche (26) und eine gegenüberliegende zweite Oberfläche (28) umfasst, die durch einen Randabschnitt (30) getrennt sind, wobei die erste Oberfläche (26) eine im Wesentlichen ebene Oberfläche (32) definiert und wobei die zweite Oberfläche (22) eine konvexe Gestalt aufweist;
wobei in Bezug auf eines oder mehrere der Stimulationselemente (24), bevorzugt in Bezug auf alle Stimulationselemente, das Stimulationselement (24) zwischen der inneren Auskleidung (12, 16, 20) aus einer Gewebelage und der äußeren Gewebelage (14, 18, 22) derart angeordnet ist, dass die erste Oberfläche (26) des Stimulationselements (24) der äußeren Gewebelage (14, 18, 22) zugewandt ist, und derart angeordnet ist, dass die zweite Oberfläche des Stimulationselements der inneren Auskleidung (12, 16, 20) aus einer Gewebelage zugewandt ist;
wobei in Bezug auf das eine oder die mehreren Stimulationselemente (24), bevorzugt in Bezug auf jedes der Stimulationselemente, die erste Oberfläche (26) des Stimulationselements unabhängig eine Fläche von 2 - 25 cm² aufweist und/oder
wobei in Bezug auf das eine oder die mehreren Stimulationselemente (24), bevorzugt in Bezug auf jedes der Stimulationselemente, der Abstand zwischen der im Wesentlichen ebenen Oberfläche (32), die durch die erste Oberfläche (26) definiert ist, und dem am weitesten entfernten Punkt (P) der zweiten Oberfläche (28) unabhängig aus den Bereichen von 1,0 - 5,5 cm ausgewählt ist.

2. Kleidungsstück (100) nach Anspruch 1, wobei das Kleidungsstück in der Form einer Weste vorliegt.

3. Kleidungsstück (100) nach Anspruch 1, wobei das Kleidungsstück zusätzlich einen rechten Ärmel, der an der ersten durchgehenden Öffnung (8) des Kleidungsstücks verbunden ist; und einen linken Ärmel, der an der zweiten durchgehenden Öffnung (10) des Kleidungsstücks verbunden ist, umfasst, wobei das Kleidungsstück (6) in der Form einer Jacke vorliegt.

4. Kleidungsstück (100) nach Anspruch 3, wobei der rechte Ärmel und/oder der linke Ärmel des Kleidungsstücks eine innere Auskleidung aus einer Gewebelage und eine äußere Gewebelage umfasst;
wobei in Bezug auf den rechten Ärmel und/oder den linken Ärmel, der/die Ärmel eine Vielzahl von Stimulationselementen (24) umfasst/umfassen;
wobei in Bezug auf eines oder mehrere der Stimulationselemente (24) des/der Ärmel(s), bevorzugt in Bezug auf alle Stimulationselemente (24) des/der Ärmel(s), das Stimulationselement eine erste Oberfläche (26) und eine gegenüberliegende zweite Oberfläche (28) umfasst, die durch einen Randabschnitt (30) getrennt sind, wobei die erste Oberfläche (26) eine im Wesentlichen ebene Oberfläche (32) definiert und wobei die zweite Oberfläche (22) eine konvexe Gestalt aufweist;
wobei in Bezug auf eines oder mehrere der Stimulationselemente (24), bevorzugt in Bezug auf alle Stimulationselemente, das Stimulationselement (24) zwischen der inneren Auskleidung aus einer Gewebelage des Ärmels und der äußeren Gewebelage des Ärmels derart angeordnet ist, dass die erste Oberfläche (26) des Stimulationselements (24) der äußeren Gewebelage des Ärmels zugewandt ist, und derart angeordnet ist, dass die zweite Oberfläche des Stimulationselements der inneren Auskleidung aus einer Gewebelage des Ärmels zugewandt ist.

5. Kleidungsstück (100) nach einem der vorhergehenden Ansprüche, wobei in Bezug auf das eine oder die mehreren Stimulationselemente (24), bevorzugt in Bezug auf jedes der Stimulationselemente, das Stimulationselement (24) an der äußeren Gewebelage (14, 18, 22) durch Anbringung mit einem Klebstoff an der ersten Oberfläche (26) des Stimulationselements (24) angebracht ist;
und/oder
wobei in Bezug auf das eine oder die mehreren Stimulationselemente (24), bevorzugt in Bezug auf jedes der Stimulationselemente, das Stimulationselement (24) an der äußeren Gewebelage (14, 18, 22) durch Anbringung mit einem Haken- und einem Schlaufenteil eines Verschlusses mit Haken- und Schlaufenteilen angebracht ist;
optional wobei die erste Oberfläche (26) des Stimulationselements (24) einen Hakenteil eines Verschlusses mit Haken- und Schlaufenteilen umfasst und wobei die äußere Gewebelage (14, 18, 22) an der Position des Stimulationselements (24) einen Schlaufenteil eines Verschlusses mit Haken- und Schlaufenteilen umfasst; oder optional wobei die erste Oberfläche (26) des Stimulationselements (24) einen Schlaufenteil eines Verschlusses mit Haken- und Schlaufenteilen umfasst und wobei die äußere Gewebelage (14, 18, 22) an der Position des Stimulationselements (24) einen Hakenteil eines Verschlusses mit Haken- und Schlaufenteilen umfasst.

6. Kleidungsstück (100) nach einem der vorhergehenden Ansprüche, wobei in Bezug auf eine Vielzahl der Stimulationselemente (24), bevorzugt in Bezug auf alle Stimulationselemente, das Stimulationselement (24) innerhalb eines Gewebeschlauchs angeordnet ist und wobei der Gewebeschlauch an der äußeren Gewebelage (14, 18, 22) befestigt ist; wobei optional der Gewebeschlauch zwischen Positionen von Stimulationselementen (24) Stiche eines Fadens umfasst, wodurch einzelne Zellen in dem Gewebeschlauch ausgebildet werden, wobei oder mehrere der Stimulationselemente in solchen Zellen eingeschlossen ist/sind.

7. Kleidungsstück (100) nach einem der vorhergehenden Ansprüche, wobei in Bezug auf den ersten vorderen Abschnitt (2), den zweiten vorderen Abschnitt (4) und/oder den hinteren Abschnitt (6) die innere Auskleidung (12, 16, 20) aus einer Gewebelage und die äußere Gewebelage (14, 18, 22) davon eine Vielzahl von eingeschlossenen Sektoren (34) ausbilden, die im Verhältnis zueinander durch Stiche (36) eines Fadens begrenzt sind, der die innere Auskleidung (12, 16, 18) aus einer Gewebelage und die äußere Gewebelage (14, 18, 22) verbindet, und wobei in Bezug auf eines oder mehrere der Stimulationselemente (24) des Teils des Kleidungsstücks, bevorzugt alle Stimulationselemente des Teils des Kleidungsstücks, das/die Stimulationselement(e) (24) innerhalb eines solchen eingeschlossenen Sektors (34) angeordnet ist/sind;
optional wobei das in Bezug auf einen oder mehrere eingeschlossene Sektoren (34) des ersten vorderen Abschnitts (2), des zweiten vorderen Abschnitts (4) und/oder des hinteren Abschnitts (6) des Kleidungsstücks das Innere des eingeschlossenen Sektors (34) eine Grundfläche (A) aufweist, gemessen als die Fläche innerhalb der Stiche (36), die den eingeschlossenen Sektor umgeben, ausgewählt ist aus den Bereichen von 6 - 100 cm², wie 10 - 95 cm², wie 15 - 90 cm², z. B. 20 - 85 cm², zum Beispiel 25 - 80 cm², wie 30 - 75 cm², wie 35 - 70 cm², zum Beispiel 40 - 65 cm², z. B. 45 - 60 cm² oder 50 - 55 cm²;
und/oder
wobei in Bezug auf einen oder mehrere eingeschlossene Sektoren (34) des ersten vorderen Abschnitts (2), des zweiten vorderen Abschnitts (4) und/oder des hinteren Abschnitts (6) die Grundfläche (A) des Inneren des begrenzten Sektors die Gestalt eines Dreiecks, eines Vierecks, wie etwa eines Rechtecks, eines Quadrats, einer Raute oder eines Sechsecks aufweist;
und/oder
wobei einer oder mehrere der eingeschlossenen Sektoren (34) unabhängig ein, zwei, drei, vier oder mehr der Stimulationselemente (24) umfasst/umfassen;
und/oder
wobei in Bezug auf einen oder mehrere der eingeschlossenen Sektoren (34), bevorzugt in Bezug auf alle eingeschlossenen Sektoren, und in Bezug auf eines oder mehrere der Stimulationselemente (24), bevorzugt in Bezug auf alle Stimulationselemente (24), die Abmessung und Geometrie des eingeschlossenen Sektors (34) derart an die Abmessung und Geometrie der darin aufgenommenen Stimulationselemente (24) angepasst wurden, dass das/die Stimulationselement(e) seine/ihre Ausrichtung nicht so ändern kann/können, dass seine/ihre erste Oberfläche beginnt, sich der inneren Auskleidung (12, 16, 20) des Kleidungsstücks zuzuwenden.

8. Kleidungsstück (100) nach einem der vorhergehenden Ansprüche, wobei in Bezug auf das eine oder die mehreren Stimulationselemente (24), bevorzugt in Bezug auf jedes der Stimulationselemente, das Stimulationselement (24) unabhängig hergestellt ist aus einem Polymer, wie etwa Gummi, Silikon oder Kunststoff; Holz; Metall oder einer Legierung, einem keramischen Material, wie etwa Glas, oder einem geschäumten Material.

9. Kleidungsstück (100) nach einem der vorhergehenden Ansprüche, wobei in Bezug auf das eine oder die mehreren der Stimulationselemente (24), bevorzugt in Bezug auf jedes der Stimulationselemente, das Stimulationselement (24) unabhängig ein Gewicht aufweist von 10 - 150 g, wie 15 - 140 g, zum Beispiel 20 - 135 g, zum Beispiel 25 - 120 g, wie 30 - 115 g, wie 35 - 110 g, z. B. 40 - 105 g, wie 45 - 100 g, zum Beispiel 50 - 95 g, wie 55 - 90 g, z. B. 60 - 85 g, wie 65 - 80 g oder 70 - 75 g;
und/oder
wobei in Bezug auf das eine oder die mehreren Stimulationselemente (24), bevorzugt in Bezug auf jedes der Stimulationselemente, die erste Oberfläche (26) des Stimulationselements unabhängig eine Fläche aufweist von 3 - 24 cm², zum Beispiel 4 - 23 cm², z. B. 5 - 22 cm², wie 6 - 21 cm², zum Beispiel 7 - 20 cm², zum Beispiel 8 - 19 cm², wie 9 - 18 cm², z. B. 10 - 17 cm², wie 11 - 18 cm², zum Beispiel 12 - 17 cm², wie 13 - 16 cm² oder 14 - 15 cm²;
und/oder
wobei in Bezug auf das eine oder die mehreren Stimulationselemente (24), bevorzugt in Bezug auf jedes der Stimulationselemente, der Abstand zwischen der im Wesentlichen ebenen Oberfläche (32), die durch die erste Oberfläche (26) definiert ist, und dem am weitesten entfernten Punkt (P) der zweiten Oberfläche (28) unabhängig aus den Bereichen von 1,5 - 5,0 cm, zum Beispiel 2,0 - 4,5 cm, wie 2,5 - 4,0 cm oder 3,0 - 3,5 cm, ausgewählt ist.

10. Kleidungsstück (100) nach einem der vorhergehenden Ansprüche, wobei die Anzahl von Stimulationselementen (24), die an dem ersten vorderen Abschnitt (2) vorhanden sind, aus den Bereichen von 5 - 40, wie etwa 10 - 35, z. B. 15 - 30, wie etwa 20 - 25, ausgewählt ist; und/oder wobei die Anzahl von Stimulationselementen (24), die an dem zweiten vorderen Abschnitt (4) vorhanden sind, aus den Bereichen von 5 - 40, wie etwa 10 - 35, z. B. 15 - 30, wie etwa 20 - 25, ausgewählt ist; und/oder wobei die Anzahl von Stimulationselementen (24), die an dem hinteren Abschnitt (6) vorhanden sind, aus den Bereichen von 10 - 85, wie etwa 15 - 80, z. B. 20 - 75, wie etwa 25 - 70, zum Beispiel 30 - 65, wie etwa 35 - 60, zum Beispiel 40 - 55 oder 45 - 50, ausgewählt ist.

11. Kleidungsstück (100) nach einem der vorhergehenden Ansprüche, wobei in Bezug auf das eine oder die mehreren Stimulationselemente (24), bevorzugt in Bezug auf jedes der Stimulationselemente, das Stimulationselement (24) unabhängig die Gestalt einer durchgeschnittenen Kugel, eines durchgeschnittenen Ellipsoids, eines durchgeschnittenen Ovoids, eines Kegels, eines Kegels mit abgerundeter Spitze, eines Tetraeders, eines Tetraeders mit abgerundeter Spitze, eines Prismas, eines Prismas mit einer abgerundeten Kante aufweist;
und/oder
wobei in Bezug auf das eine oder die mehreren Stimulationselemente (24), bevorzugt in Bezug auf jedes der Stimulationselemente, die erste Oberfläche (26) des Stimulationselements (24) eine im Wesentlichen ebene Oberfläche ist;
und/oder
wobei in Bezug auf eines oder mehrere der Stimulationselemente (24) die erste Oberfläche (26) des Stimulationselements (24) einen Hohlraum (46) umfasst.

12. Kleidungsstück (100) nach einem der vorhergehenden Ansprüche, wobei in Bezug auf den ersten vorderen Abschnitt (2), den zweiten vorderen Abschnitt (4) und/oder den hinteren Abschnitt (6) des Kleidungsstücks das Kleidungsstück zusätzlich eine Auspolsterungslage umfasst, die zwischen einer inneren Oberfläche der äußeren Gewebelage (14, 18, 22) und der im Wesentlichen flachen Oberfläche (32) der ersten Oberfläche (26) der Stimulationselemente (24) angeordnet ist, wie etwa an einer äußeren Oberfläche der inneren Auskleidung (12, 16, 20) aus einer Gewebelage.

13. Kleidungsstück (100) nach einem der vorhergehenden Ansprüche, wobei in Bezug auf den ersten vorderen Abschnitt (2) und/oder den zweiten vorderen Abschnitt (4) der Abschnitt zusätzlich eine oder mehrere Taschen (44) umfasst.

14. Kleidungsstück (100) nach einem der vorhergehenden Ansprüche, wobei das Kleidungsstück einen Reißverschluss (42) umfasst, der entlang einer Kante (38) des ersten vorderen Abschnitts (2) und entlang einer Kante (30) des zweiten vorderen Abschnitts (4) angeordnet ist, um den ersten vorderen Abschnitt (2) an den/von dem zweiten vorderen Abschnitt (4) mittels Reißverschluss zu koppeln/zu lösen; oder
wobei das Kleidungsstück einen oder mehrere Knöpfe und ein oder mehrere entsprechende Knopflöcher umfasst, wobei der eine oder die mehreren Knöpfe entlang einer Kante (38) des ersten vorderen Abschnitts (2) des Kleidungsstücks angeordnet sind und wobei das eine oder die mehreren Knopflöcher entlang einer Kante (40) des zweiten vorderen Abschnitts (4) des Kleidungsstücks angeordnet sind; oder wobei das eine oder die mehreren Knopflöcher entlang einer Kante (38) des ersten vorderen Abschnitts (2) des Kleidungsstücks angeordnet sind und wobei der eine oder die mehreren Knöpfe entlang einer Kante (40) des zweiten vorderen Abschnitts (4) des Kleidungsstücks angeordnet sind.

15. Kleidungsstück (100) nach einem der Ansprüche 1 - 14 zur Verwendung bei der Therapie gegen psychiatrische Krankheiten; wobei die psychiatrische Krankheit optional aus der Gruppe von Folgenden ausgewählt ist: Aufmerksamkeitsdefizit- und Hyperaktivitätsstörung (ADHS), sensorische Verarbeitungsstörung (SPD), Autismus-Spektrum-Störungen, Depression, Angst, Rastlosigkeit, Demenz, kognitive Störungen.

## Revendications

1. Vêtement (100) pour fournir une stimulation sensorielle tactile au torse d'un utilisateur, dans lequel ledit vêtement comprend :
- une première partie avant (2) ;
- une seconde partie avant (4) ;
- une partie arrière (6) ;
dans lequel la première partie avant (2) est reliée à la partie arrière (6) de telle manière qu'une première ouverture traversante (8) pour un bras droit soit présente dans le vêtement ;
dans lequel la seconde partie avant (4) est reliée à la partie arrière (6) de telle manière qu'une seconde ouverture traversante (10) pour un bras gauche soit présente dans le vêtement ;
dans lequel la première partie avant (2) comprend une doublure intérieure (12) d'une couche de tissu et une couche de tissu extérieure (14) ;
dans lequel la seconde partie avant (4) comprend une doublure intérieure (16) d'une couche de tissu et une couche de tissu extérieure (18) ;
dans lequel la partie arrière (6) comprend une doublure intérieure (20) d'une couche de tissu et une couche de tissu extérieure (22) ;
dans lequel, en ce qui concerne la première partie avant (2), la seconde partie avant (4) et la partie arrière (6), ladite partie comprend une pluralité d'éléments de stimulation (24) ;
**caractérisé en ce que**, en ce qui concerne un ou plusieurs desdits éléments de stimulation (24), de préférence en ce qui concerne tous lesdits éléments de stimulation (24), ledit élément de stimulation comprend une première surface (26) et une seconde surface opposée (28) séparées par une partie de rebord (30), dans lequel ladite première surface (26) définit une surface essentiellement plate (32) et dans lequel ladite seconde surface (22) possède une forme convexe ;
dans lequel, en ce qui concerne un ou plusieurs desdits éléments de stimulation (24), de préférence en ce qui concerne tous lesdits éléments de stimulation, ledit élément de stimulation (24) est agencé entre ladite doublure intérieure (12, 16, 20) de la couche de tissu et ladite couche de tissu extérieure (14, 18, 22) de telle manière que ladite première surface (26) dudit élément de stimulation (24) fasse face à ladite couche de tissu extérieure (14, 18, 22), et de telle manière que ladite seconde surface dudit élément de stimulation fasse face à ladite doublure intérieure (12, 16, 20) de la couche de tissu ;
dans lequel en ce qui concerne lesdits un ou plusieurs éléments de stimulation (24), de préférence en ce qui concerne chacun desdits éléments de stimulation, ladite première surface (26) dudit élément de stimulation possède indépendamment une aire de 2 à 25 cm² et/ou
dans lequel en ce qui concerne lesdits un ou plusieurs éléments de stimulation (24), de préférence en ce qui concerne chacun desdits éléments de stimulation, la distance entre ladite surface essentiellement plate (32) définie par ladite première surface (26) et le point le plus éloigné (P) de ladite seconde surface (28) est sélectionnée indépendamment dans les plages de 1,0 *à 5,5* cm.

2. Vêtement (100) selon la revendication 1, dans lequel ledit vêtement se présente sous la forme d'un gilet.

3. Vêtement (100) selon la revendication 1, dans lequel ledit vêtement comprend en outre une manche droite qui est reliée à ladite première ouverture traversante (8) dudit vêtement ; et une manche gauche qui est reliée à ladite seconde ouverture traversante (10) dudit vêtement, dans lequel ledit vêtement (6) a la forme d'une veste.

4. Vêtement (100) selon la revendication 3, dans lequel ladite manche droite et/ou ladite manche gauche dudit vêtement comprend une doublure intérieure d'une couche de tissu et une couche de tissu extérieure ;
dans lequel, en ce qui concerne la manche droite et/ou la manche gauche, ladite ou lesdites manches comprennent une pluralité d'éléments de stimulation (24) ;
dans lequel en ce qui concerne un ou plusieurs desdits éléments de stimulation (24) de ladite ou desdites manches, de préférence en ce qui concerne tous lesdits éléments de stimulation (24) de ladite ou desdites manches, ledit élément de stimulation comprend une première surface (26) et une seconde surface opposée (28) séparées par une partie de rebord (30), dans lequel ladite première surface (26) définit une surface essentiellement plate (32) et dans lequel ladite seconde surface (22) possède une forme convexe ;
dans lequel, en ce qui concerne un ou plusieurs desdits éléments de stimulation (24), de préférence en ce qui concerne tous lesdits éléments de stimulation, ledit élément de stimulation (24) est agencé entre ladite doublure intérieure de couche de tissu de ladite manche et ladite couche de tissu extérieure de ladite manche de telle manière que ladite première surface (26) dudit élément de stimulation (24) fasse face à ladite couche de tissu extérieure de ladite manche, et de telle manière que ladite seconde surface dudit élément de stimulation fasse face à ladite doublure intérieure de la couche de tissu de ladite manche.

5. Vêtement (100) selon l'une quelconque des revendications précédentes, dans lequel, en ce qui concerne lesdits un ou plusieurs éléments de stimulation (24), de préférence en ce qui concerne chacun desdits éléments de stimulation, ledit élément de stimulation (24) est fixé à ladite couche de tissu extérieure (14, 18, 22) par fixation par un adhésif à ladite première surface (26) dudit élément de stimulation (24) ;
et/ou
dans lequel, en ce qui concerne lesdits un ou plusieurs éléments de stimulation (24), de préférence en ce qui concerne chacun desdits éléments de stimulation, ledit élément de stimulation (24) est fixé à ladite couche de tissu extérieure (14, 18, 22) par fixation par des parties de ruban autoagrippant d'une attache à ruban autoagrippant ;
éventuellement, dans lequel ladite première surface (26) dudit élément de stimulation (24) comprend une partie à crochets d'une attache à ruban autoagrippant, et dans lequel ladite couche de tissu extérieure (14, 18, 22), à la position dudit élément de stimulation (24) comprend une partie à boucles d'une attache à ruban autoagrippant ; ou éventuellement, dans lequel ladite première surface (26) dudit élément de stimulation (24) comprend une partie à boucles d'une attache à ruban autoagrippant, et dans lequel ladite couche de tissu extérieure (14, 18, 22), à la position dudit élément de stimulation (24) comprend une partie à crochets d'une attache à ruban autoagrippant.

6. Vêtement (100) selon l'une quelconque des revendications précédentes, dans lequel, en ce qui concerne une pluralité desdits éléments de stimulation (24), de préférence en ce qui concerne tous lesdits éléments de stimulation, ledit élément de stimulation (24) est agencé à l'intérieur d'un tube de tissu et dans lequel ledit tube de tissu est fixé à ladite couche de tissu extérieure (14, 18, 22) ; éventuellement dans lequel ledit tube de tissu, entre les positions des éléments de stimulation (24), comprend des mailles d'un fil, formant ainsi des cellules individuelles dans ledit tube de tissu, dans lequel ou plusieurs desdits éléments de stimulation est/sont confiné(s) dans de telles cellules.

7. Vêtement (100) selon l'une quelconque des revendications précédentes, dans lequel, en ce qui concerne ladite première partie avant (2), ladite seconde partie avant (4) et/ou ladite partie arrière (6), ladite doublure intérieure (12, 16, 20) d'une couche de tissu et ladite couche de tissu extérieure (14, 18, 22) de celle-ci forment une pluralité de secteurs confinés (34) qui sont délimités les uns par rapport aux autres par des mailles (36) de fil qui relie ladite doublure intérieure (12, 16, 18) d'une couche de tissu et ladite couche de tissu extérieure (14, 18, 22) et dans lequel, en ce qui concerne un ou plusieurs desdits éléments de stimulation (24) de ladite partie du vêtement, de préférence tous lesdits éléments de stimulation de ladite partie du vêtement, ledit ou lesdits éléments de stimulation (24) est/sont agencé(s) à l'intérieur d'un tel secteur confiné (34) ;
éventuellement dans lequel le, en ce qui concerne un ou plusieurs secteurs confinés (34) de ladite première partie avant (2), de ladite seconde partie avant (4) et/ou de ladite partie arrière (6) dudit vêtement, ledit intérieur dudit secteur confiné (34) possède une aire de base (A), telle que mesurée comme l'aire à l'intérieur desdites mailles (36) entourant ledit secteur confiné, est choisie dans les plages de 6 à 100 cm², telles que 10 à 95 cm², telles que 15 à 90 cm², par exemple 20 à 85 cm², par exemple 25 à 80 cm², telles que 30 à 75 cm², telles que 35 à 70 cm², par exemple 40 à 65 cm², par exemple 45 à 60 cm² ou 50 à 55 cm² ;
et/ou
dans lequel, en ce qui concerne un ou plusieurs secteurs confinés (34) de ladite première partie avant (2), de ladite seconde partie avant (4) et/ou de ladite partie arrière (6), la zone de base (A) dudit intérieur dudit secteur confiné possède la forme d'un triangle, d'un tétragone, tel qu'un rectangle, un carré, un losange ou un hexagone ;
et/ou
dans lequel un ou plusieurs desdits secteurs confinés (34) comprend/comprennent indépendamment un, deux, trois, quatre ou plus desdits éléments de stimulation (24) ;
et/ou
dans lequel, en ce qui concerne un ou plusieurs desdits secteurs confinés (34), de préférence en ce qui concerne tous lesdits secteurs confinés, et en ce qui concerne un ou plusieurs desdits éléments de stimulation (24), de préférence en ce qui concerne tous les éléments de stimulation (24), la dimension et la géométrie dudit secteur confiné (34) ont été adaptées à la dimension et à la géométrie desdits éléments de stimulation (24) qui y sont logés de telle manière que ledit ou lesdits éléments de stimulation ne puisse/puissent pas changer son/leur orientation de sorte que sa/leur première surface soit d'abord orientée vers la doublure intérieure (12, 16, 20) dudit vêtement.

8. Vêtement (100) selon l'une quelconque des revendications précédentes, dans lequel, en ce qui concerne lesdits un ou plusieurs éléments de stimulation (24), de préférence en ce qui concerne chacun desdits éléments de stimulation, ledit élément de stimulation (24) est fabriqué indépendamment à partir d'un polymère, tel que du caoutchouc, silicone ou plastique ; bois ; métal ou un alliage, un matériau céramique, tel que le verre, ou un matériau en mousse.

9. Vêtement (100) selon l'une quelconque des revendications précédentes, dans lequel, en ce qui concerne lesdits un ou plusieurs desdits éléments de stimulation (24), de préférence en ce qui concerne chacun desdits éléments de stimulation, ledit élément de stimulation (24) possède indépendamment un poids de 10 à 150 g, tel que 15 à 140 g, par exemple 20 à 135 g, par exemple 25 à 120 g, tel que 30 à 115 g, tel que 35 à 110 g, par exemple 40 à 105 g, tel que 45 à 100 g, par exemple 50 à 95 g, tel que 55 à 90 g, par exemple 60 à 85 g, tel que 65 à 80 g ou 70 à 75 g ;
et/ou
dans lequel en ce qui concerne lesdits un ou plusieurs éléments de stimulation (24), de préférence en ce qui concerne chacun desdits éléments de stimulation, ladite première surface (26) dudit élément de stimulation possède indépendamment une aire de 3 à 24 cm², par exemple 4 à 23 cm², par exemple 5 à 22 cm², comme 6 à 21 cm², par exemple 7 à 20 cm², par exemple 8 à 19 cm², comme 9 à 18 cm², par exemple 10 à 17 cm², comme 11 à 18 cm², par exemple 12 à 17 cm², comme 13 à 16 cm² ou 14 à 15 cm² ;
et/ou
dans lequel en ce qui concerne lesdits un ou plusieurs éléments de stimulation (24), de préférence en ce qui concerne chacun desdits éléments de stimulation, la distance entre ladite surface essentiellement plate (32) définie par ladite première surface (26) et le point le plus éloigné (P) de ladite seconde surface (28) est sélectionnée indépendamment dans les plages de 1,5 à 5,0 cm, par exemple 2,0 à 4,5 cm, telles que 2,5 à 4,0 cm ou 3,0 à 3,5 cm.

10. Vêtement (100) selon l'une quelconque des revendications précédentes, dans lequel le nombre d'éléments de stimulation (24) présents au niveau de ladite première partie avant (2) est choisi dans les plages de 5 à 40, telles que 10 à 35, par exemple 15 à 30, telles que 20 à 25 ; et/ou dans lequel le nombre d'éléments de stimulation (24) présents au niveau de ladite seconde partie avant (4) est choisi dans les plages de 5 à 40, telles que 10 à 35, par exemple 15 à 30, telles que 20 à 25 ; et/ou dans lequel le nombre d'éléments de stimulation (24) présents au niveau de ladite partie arrière (6) est choisi dans les plages de 10 à 85, telles que 15 à 80, par exemple 20 à 75, telles que 25 à 70, par exemple 30 à 65, telles que 35 à 60, par exemple 40 à 55 ou 45 à 50.

11. Vêtement (100) selon l'une quelconque des revendications précédentes, dans lequel, en ce qui concerne lesdits un ou plusieurs éléments de stimulation (24), de préférence en ce qui concerne chacun desdits éléments de stimulation, l'élément de stimulation (24) possède indépendamment la forme d'une sphère coupée, d'un ellipsoïde coupé, d'un ovoïde coupé, d'un cône, d'un cône à sommet arrondi, d'un tétraèdre, d'un tétraèdre à sommet arrondi, d'un prisme, d'un prisme à bord arrondi ;
et/ou
dans lequel en ce qui concerne lesdits un ou plusieurs éléments de stimulation (24), de préférence en ce qui concerne chacun desdits éléments de stimulation, la première surface (26) dudit élément de stimulation (24) est une surface essentiellement plate ;
et/ou
dans lequel, en ce qui concerne un ou plusieurs desdits éléments de stimulation (24), ladite première surface (26) dudit élément de stimulation (24) comprend une cavité (46).

12. Vêtement (100) selon l'une quelconque des revendications précédentes, dans lequel, en ce qui concerne ladite première partie avant (2), ladite seconde partie avant (4) et/ou ladite partie arrière (6) dudit vêtement, ledit vêtement comprend en outre une couche de rembourrage qui est agencée entre une surface intérieure de la couche de tissu extérieure (14, 18, 22) et ladite surface essentiellement plate (32) de ladite première surface (26) desdits éléments de stimulation (24), comme au niveau d'une surface extérieure de ladite doublure intérieure (12, 16, 20) d'une couche de tissu.

13. Vêtement (100) selon l'une quelconque des revendications précédentes, dans lequel, en ce qui concerne ladite première partie avant (2) et/ou ladite seconde partie avant (4), ladite partie comprend en outre une ou plusieurs poches (44).

14. Vêtement (100) selon l'une quelconque des revendications précédentes, dans lequel ledit vêtement comprend une fermeture à glissière (42) agencée le long d'un bord (38) de la première partie avant (2) et le long d'un bord (30) de la seconde partie avant (4) pour fermer/ouvrir ladite première partie avant (2) et ladite seconde partie avant (4) ; ou
dans lequel ledit vêtement comprend un ou plusieurs boutons et une ou plusieurs boutonnières correspondantes, dans lequel lesdits un ou plusieurs boutons sont agencés le long d'un bord (38) de la première partie avant (2) dudit vêtement et dans lequel lesdites une ou plusieurs boutonnières sont agencées le long d'un bord (40) de la seconde partie avant (4) dudit vêtement ; ou dans lequel lesdites une ou plusieurs boutonnières sont agencées le long d'un bord (38) de la première partie avant (2) dudit vêtement et dans lequel lesdits un ou plusieurs boutons sont agencés le long d'un bord (40) de la seconde partie avant (4) dudit vêtement.

15. Vêtement (100) selon l'une quelconque des revendications 1 à 14, destiné à être utilisé en thérapie contre des états psychiatriques ; dans lequel l'état psychiatrique est éventuellement choisi dans le groupe constitué de : trouble déficitaire de l'attention avec hyperactivité (THADA), trouble du traitement sensoriel (TSP), troubles appartenant au spectre de l'autisme, dépression, anxiété, agitation, démence, troubles cognitifs.
